(19)

Europäisches
Patentamt
European
Patent Office
Office européen
des brevets

(11) **EP 3 941 358 B1**

(12) **EUROPEAN PATENT SPECIFICATION**

(45) Date of publication and mention
of the grant of the patent:
**05.03.2025 Bulletin 2025/10**

(21) Application number: **20712932.1**

(22) Date of filing: **19.03.2020**

(51) International Patent Classification (IPC):
*A61B 8/06* *(2006.01)*    *A61B 8/08* *(2006.01)*
*A61B 8/13* *(2006.01)*    *A61B 8/00* *(2006.01)*
*G01S 15/89* *(2006.01)*

(52) Cooperative Patent Classification (CPC):
**A61B 8/06; A61B 8/0891; A61B 8/13; A61B 8/483;
A61B 8/488; A61B 8/5223; A61B 8/543;
G01S 15/8993;** G01S 15/8925; G01S 15/8934

(86) International application number:
**PCT/EP2020/057584**

(87) International publication number:
**WO 2020/188022 (24.09.2020 Gazette 2020/39)**

(54) **THREE DIMENSIONAL VOLUME FLOW QUANTIFICATION AND MEASUREMENT**

QUANTIFIZIERUNG UND MESSUNG DREIDIMENSIONALER VOLUMENSTRÖME

QUANTIFICATION ET MESURE DE DÉBIT VOLUMIQUE TRIDIMENSIONNEL

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **19.03.2019 US 201962820549 P**

(43) Date of publication of application:
**26.01.2022 Bulletin 2022/04**

(73) Proprietor: **Koninklijke Philips N.V.
5656 AG Eindhoven (NL)**

(72) Inventors:
• **HUANG, Sheng-Wen
5656 AE Eindhoven (NL)**
• **JAGO, James Robertson
5656 AE Eindhoven (NL)**
• **LI, Sibo
5656 AE Eindhoven (NL)**
• **WANG, Shiying
5656 AE Eindhoven (NL)**
• **SHIN, Jun Seob
5656 AE Eindhoven (NL)**
• **HARRISON, Gerard Joseph
5656 AE Eindhoven (NL)**
• **LOUPAS, Thanasis
5656 AE Eindhoven (NL)**
• **ZHANG, Liang
5656 AE Eindhoven (NL)**

(74) Representative: **Philips Intellectual Property &
Standards
High Tech Campus 52
5656 AG Eindhoven (NL)**

(56) References cited:
WO-A1-2019/156975    US-A1- 2008 287 799
US-A1- 2010 249 597    US-A1- 2012 123 267
US-B1- 6 503 202

**Description**

This invention relates to medical diagnostic ultrasound systems and, in particular, to ultrasound systems which produce quantified measurements of the volume flow of blood through the heart or a blood vessel.

Ultrasound has long been used to assess various parameters of blood flow in the heart and vascular system using the Doppler principle. The basic Doppler response is flow velocity, which can further be used to determine additional characteristics of blood flow. One characteristic of interest to cardiologists is the volume flow of blood through a vessel. Early efforts to estimate volume flow consisted of multiplying the mean velocity of blood flow by the nominal cross-sectional area of a blood vessel. However, these early efforts had shortcomings due to the need to make certain estimates. One is that the vessel lumen is circular. Another is the estimation of the mean velocity from a single Doppler measurement or from a qualitative assessment of spectral Doppler data. Velocity measurement must also be corrected for the angle between the ultrasound beam direction and the direction of flow. Yet another consideration is the laminar flow profile in the presence of stenosis.

A further complication arises due to the pulsatility of arterial flow. While venous flow is substantially constant, arterial flow is constantly changing over the heart cycle. Thus, the standard techniques often lack for user independency and repeatability. Some of these demands have been eased by the advent of 3D ultrasound to assess flow conditions and particularly its ability to acquire volume blood flow information. With 3D imaging, the full vessel lumen can be imaged and a sequence of 3D image data sets acquired for later replay and diagnosis. When data of the full volumetric flow in the vessel is acquired in the data sets, the image data can be examined during post-acquisition diagnosis to assess the flow profile. Different 2D image planes can be extracted from the 3D data in multi-planar reconstruction (MPR), so that an image plane of a desired orientation through a vessel can be examined. Three dimensional imaging thus addresses many of the static imaging challenges which are problematic with 2D flow estimation.

In recent years the problem of analyzing the temporal dynamics of blood flow have been addressed by a technique called "spatial-temporal image correlation," or STIC. With STIC, a sweep is made through a blood vessel with ultrasound and many image frames are acquired over a sequence of heart cycles. When done by manually scanning with a 2D ultrasound probe, this image acquisition can take ten seconds or longer. The same acquisition can be performed with a mechanical 3D probe which mechanically sweeps the image plane through the vessel, but 3D mechanical probes often have poorer elevation focus which leads to inaccuracies when constructing MPR images in the elevation dimension. After the acquisition is complete and the image frames are stored, image frames of the desired anatomy, created by MPR reconstruction if necessary, are reassembled into a loop of images according to their phase sequence in the cardiac cycle. This task is made difficult by the fact that the heart cycle may not be uniform over the time required to acquire the data sets. Consequently, synthetic methods of estimating the heart rate from analysis of the movement of cardiac tissue or blood have been developed, which nonetheless is often difficult to assess and prone to inaccuracy. Accordingly, it is desirable to develop more robust techniques for accurately assessing volume flow in the presence of flow pulsatility and erratic heartbeats. It is further desirable to automate such techniques so as to prioritize and shorten the time interval needed to acquire the volume flow data and reduce the impact of motional effects by both the probe and the anatomy.

US 2008/287799 A1 discloses an ultrasound system comprising an ultrasound probe, a user interface and a processor. The ultrasound probe comprises a transducer face emitting ultrasound beams into a patient. The probe acquires a volume of ultrasound data comprising a blood vessel. The user interface defines a surface on an image that is based on the volume. The surface bisects the blood vessel and further comprises a plurality of points where at least some of the points are located at unequal distances with respect to the transducer face. The processor is configured to steer a subset of the ultrasound beams to intersect the surface at a 90 degree angle and calculate volumetric flow information through the blood vessel based on the ultrasound data corresponding to the surface.

US 6,503,202 B1 discloses a medical diagnostic ultrasound method and a system for automated flow analysis in which multiple cross-sectional areas along a vessel are determined automatically. A processor locates an abnormality as a function of the multiple cross-sectional areas, such as identifying a cross-sectional area that is a threshold amount less than an average cross-sectional area. The abnormal area is highlighted on the display to assist with medical diagnosis.

WO 2019/156975 A1 discloses an ultrasound imaging system for guiding a user to acquire ultrasound image data sets that can be analyzed for the presence of atherosclerosis. The system comprises a processor programmed to analyze ultrasound image data for a vessel of interest and to control a user interface that suggests how an operator should move a transducer to acquire ultrasound image data with the vessel of interest in a desired location. The processor stores multiple ultrasound image data sets taken along a length of the vessel of interest to be analyzed for the presence of an atheroma.

The invention is defined by the independent claims. Advantageous embodiments are provided in the dependent claims.

In accordance with the principles of the present invention, a diagnostic ultrasound system is described which produces a blood flow profile using volume flow acquisition. A 3D imaging probe is used to acquire one or more image volumes of flow data from a vessel and a B- or C- cross-sectional plane of the vessel is extracted which is processed to determine the blood volume flow rate. When arterial flow is being assessed, it is preferable to acquire multiple subvolumes of the volume flow, with each spatially different volume flow dataset being acquired over all phases of a cardiac cycle to maintain temporal sampling precision. The subvolumes are then spatially assembled in cardiac phase order so as to produce a full volume flow sequence with adequate temporal sampling. The B- or C-plane is then extracted and a volume flow profile estimated through the plane using Gauss's theorem.

Volume or subvolume acquisition starts around the center of the vessel where flow signals are stronger and thus cardiac phases are easier to identify. This also results in acquisition of subvolumes with the greatest contribution to total volume flow earlier in the acquisition process to minimize adverse motional effects. The acquisition process will begin by identifying the vessel center prior to subvolume acquisition, as by user designation or an automated technique such as a rapid Doppler sequence through the vessel. Subvolume acquisition then begins from the vessel center and proceeds outward therefrom.

Acquisition in synchronism with the phase of the heart cycle can be achieved by a non-synthetic method of measuring the heart cycle (e.g., an ECG monitor attached to the patient), or by a synthetic method such as user estimation or automatic M-mode or speckle tracking of cardiac or vascular anatomy or blood flow. Preferably the flow profile is calculated on the fly during acquisition, and heart rate estimations based on the cardiac cycles of previously acquired subvolumes are updated each heart cycle to account for irregularities in the heart cycle. Preferably the acquisition of each subvolume is temporally centered about the systolic phase, so that systolic flow, when volume flow is greatest in arterial vessels, is fully sampled.

According to aspects of the invention, an ultrasonic diagnostic imaging system for analyzing volume flow of blood is provided in claim 1. The system includes a 3D imaging probe adapted to acquire volume image flow data sets of a blood vessel, an image data processor responsive to the volume image flow data sets, and a vessel center locator. The vessel center locator is responsive to spatially organized blood vessel data, which is adapted to identify a center of the blood vessel. The system further includes a beamformer controller, responsive to the vessel center locator, which is adapted to control the 3D imaging probe to acquire volume image flow data sets of the blood vessel commencing around the center of the vessel. There is also a volume flow calculator, which is responsive to acquired volume image flow data sets of the blood vessel, which is adapted to calculate volume flow profile data.

The ultrasonic diagnostic imaging system further includes a heart rate calculator adapted to produce data representing a heart rate and the volume image flow data sets are acquired in timed relation to the heart rate data.

The heart rate calculator is further coupled to the volume flow calculator and adapted to calculate a subvolume acquisition time in relation to systolic peaks of a flow profile.

In some embodiments, the heart rate calculator includes one of an ECG monitor or an ultrasound data processor which is adapted to produce estimated heart rate data using ultrasound data.

In some embodiments, the ultrasonic diagnostic imaging system includes a subvolume selector responsive to the vessel center locator. The subvolume selector controls the beamformer controller to acquire volume image flow data sets of subvolumes of a blood vessel commencing around the center of the vessel.

In some embodiments, the beamformer controller acquires volume image flow data sets of a subvolume of a blood vessel over the duration of a heart cycle.

In some embodiments, the beamformer controller acquires volume image flow data sets of a subvolume of a blood vessel over an acquisition interval commencing in the middle of a diastolic portion of a heart cycle and ending in the middle of the next diastolic portion of a heart cycle.

In some embodiments, the beamformer controller acquires volume image flow data sets of a subvolume of a blood vessel during systolic heart phases occurring in the middle of the acquisition interval.

In some embodiments, the ultrasonic diagnostic imaging system further includes a 3D image data memory adapted to store volume image data sets. In some embodiments, the ultrasonic diagnostic imaging system further includes a multi-planar reformatter, which is coupled to the 3D image data memory and selects an image plane intersecting the blood vessel.

In some embodiments, the volume flow calculator is further adapted to calculate volume flow profile data in relation to the image plane intersecting the blood vessel.

In some embodiments, the system further includes a volume renderer coupled to the 3D image data memory and adapted to produce a 3D image of the blood vessel.

In some embodiments, the system further includes a display configured to display one or more of an image plane selected by the multi-planar reformatter, a 3D image of the blood vessel, and a flow profile curve. The display may be coupled to one or more other elements of the system.

The present invention also provides a method of analyzing volume flow of blood by ultrasound data acquisition as defined in claim 10, the method comprising: identifying a center of a blood vessel; acquiring volume image flow data sets of the blood vessel from subvolumes of the vessel starting with a subvolume from the center of the vessel with a 3D ultrasound imaging probe; processing image data sets acquired with the imaging probe for the display of an ultrasound image of the blood vessel; and calculating volume flow profile data using the acquired volume image flow data sets.

The method also includes estimating heart cycle timing. Volume image flow data sets of the blood vessel are acquired from subvolumes of the vessel in synchronism with the heart cycle timing, wherein a subvolume acquisition time is calculated in relation to systolic peaks of a flow profile.

In some embodiments, the method includes detecting a systolic phase of the heart cycle. In some embodiments, the volume image flow data sets of the blood vessel are acquired from a subvolume of the vessel during an acquisition interval starting in mid-diastole of a heart cycle and ending in mid-diastole of a subsequent heart cycle, wherein acquisition during the systolic phase occurs during the middle of the acquisition interval.

In some embodiments, acquiring the volume image flow data sets of the blood vessel from subvolumes of the vessel further comprises updating the estimated heart cycle timing during a plurality of the subvolume acquisition intervals.

In the drawings:

> FIGURE 1 illustrates the acquisition of a subvolume of ultrasound data of a volumetric region using a 3D ultrasonic imaging probe.
> FIGURE 2 illustrates a sequence of acquisition of subvolumes of ultrasound data of a blood vessel lumen which starts from the center of the vessel.
> FIGURE 3 illustrates a typical series of flow profiles of the acquisition sequence of FIGURE 2 when each subvolume acquisition is temporally centered about the systolic phase.
> FIGURE 4 illustrates how volume flow overestimation or under-estimation can arise when the heart cycle is inaccurately measured or estimated.
> FIGURE 5 illustrates the determination of a subsequent subvolume acquisition interval from the sampling of an initial subvolume over two heart cycles.
> FIGURES 6 illustrates the determination of a subsequent subvolume acquisition interval when sampling of the initial subvolume begins during systole.
> FIGURE 7 illustrates in block diagram form an ultrasonic diagnostic imaging system constructed in accordance with the principles of the present invention.

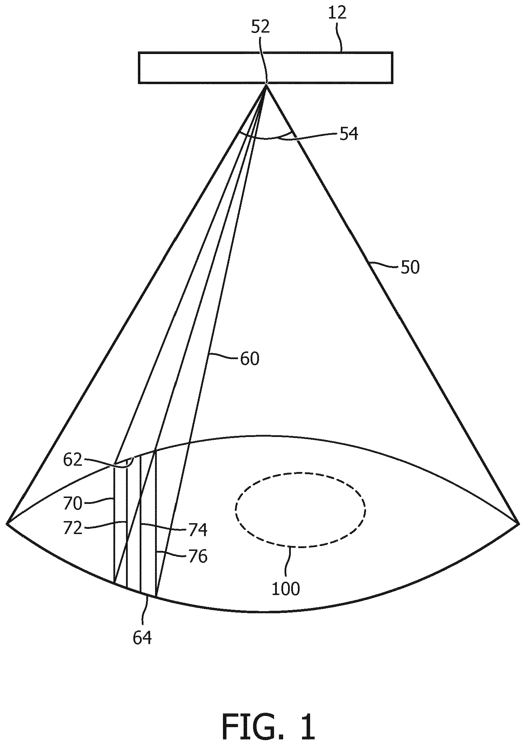

FIGURE 1 illustrates the acquisition of a subvolume 60 of ultrasound data of a volumetric region 50 by the transducer 12 of a 3D ultrasonic imaging probe. In this example the full volume 50 has a conical shape subtending a sector angle 54 with an apex 52 located at the front surface of an array transducer 12. For subvolume scanning the full volume may be scanned by acquiring ultrasound data from a series of adjacent subvolumes 60.

Each subvolume may comprise a series of two-dimensional sector-shaped slices 70, 72, 74 and 76, where each slice comprising a series of adjacent scanlines. While the 3D probe may comprise an oscillating onedimensional transducer array, preferably the probe comprises a stationary two-dimensional matrix array transducer for improved scanning speed and accuracy. Since beams of a matrix array are electronically steered in both elevation and azimuth, the full volume and the individual subvolumes may have any desired size and shape, and the slices of a subvolume can be scanned in any beam order sequence. The illustration of FIGURE 1 also shows a dashed outline 100 where a blood vessel lumen intersects the base of the volume 50 between the front and back edges of the volume 64 and 62.

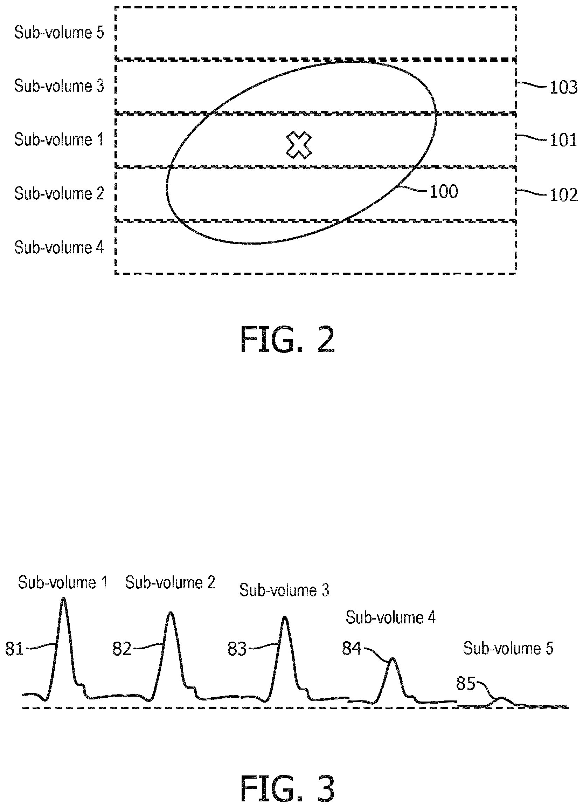

It is preferable to acquire subvolume data of a vessel lumen beginning in the center of the lumen where flow signals are strongest and it is easier to reliably identify cardiac phases. More flow signals are contained in a central subvolume and preserved after wall filtering for analysis such as heart rate estimation as discussed below. Central subvolumes also comprise the greatest contributions to total volume flow of the vessel. Such an acquisition sequence is illustrated in FIGURE 2 where subvolumes 1-5 are acquired of a vessel lumen 100 starting with a subvolume from the center of the vessel. The vessel center may be identified by user designation thereof. For instance, the user can view a B mode image of the blood vessel, which shows the vessel wall. The user clicks a cursor at what appears to be the center of the vessel, and this identification is used by the ultrasound system to demarcate the region where acquisition commences. Automated techniques can alternatively be used. For instance, prior to acquisition a series of Doppler beams are transmitted and received from the entire blood vessel and Doppler processed to produce velocity estimates along all of the beams. The spatial location on a beam with the highest velocity may then be used as demarcating the vessel center, and subvolume acquisition then proceeds from this location outward. The vessel center acquired by one of these or another technique is marked with an "X" in FIGURE 2. Acquisition then commences with the acquisition of subvolume 1 which includes the vessel center. Temporally different acquisitions of flow (velocity) data of the subvolume are performed for a complete set of phases of the heart cycle. The acquisition of subsequent subvolumes then continues outward from the central location, including subvolumes 2, 3, 4 and 5 in this example. As can be seen from the drawing, the initially acquired subvolume includes the greatest amount of flow data, and the peripheral subvolumes 4 and 5 contain the smallest contributions to the total volume flow of the vessel.

FIGURE 3 illustrates these differences in volume flow content, represented by the amplitudes of the flow signal levels of the different subvolumes. The flow signal 81 of the first subvolume is seen to exhibit the greatest amplitude, with the flow signals 82 and 83 of subvolumes 2 and 3 being of slightly lesser amplitudes. The peripheral subvolumes 4 and 5 exhibit flow signals 84 and 85 with the smallest amplitude and hence the smallest contributions to total volume flow, as well as the greatest possibility of flow phase ambiguity. It is for this reason, as well as motional effect minimization, that data from these peripheral subvolumes are the last to be acquired.

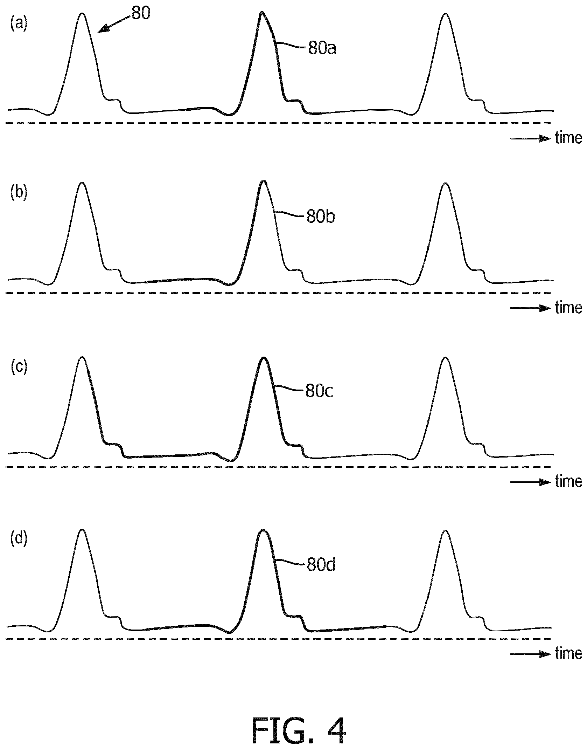

It is also seen in FIGURE 3 that the systolic phases, where the flow velocity peaks at peak systole, is in the temporal middle of each subvolume acquisition interval. This sampling timing advantageously results in complete acquisition of flow during systole when volume flow is the greatest. Inaccuracies that can occur with other heart phase timings are illustrated in FIGURE 4, which shows flow profiles 80 of four differently phased acquisitions. FIGURE 4a illustrates a situation where the estimation of the heart rate is greater than the actual heart rate. Since the heart rate is estimated to be more rapid than the actual heart rate, the interval of subvolume acquisition is shorter than it should be, as illustrated by the portion 80a of the profile shown in bold. Consequently, the acquisition interval omits some of the phases of diastole, and the resulting data is overly dominated by greater systolic flow. As a result, the volume flow rate will be over-estimated for the subvolume. Overestimation of the heart rate can also result in the situation of FIGURE 4b, where acquisition during the interval of 80b misses some systolic phase information, in this example, the second half of systole. As a result, the volume flow rate is under-estimated for the subvolume. FIGURE 4c shows a situation where the heart rate is estimated to be less (slower) than the actual heart rate. As a result, an acquisition interval will be greater than one heart cycle. In this example systole is oversampled when the subvolume is sampled during the acquisition interval of 80c, and volume flow is over-estimated. FIGURE 4d illustrates another situation where the heart rate is estimated to be less than the actual heart rate. The subvolume is sampled during the acquisition interval of bold flow profile line 80d. In this case, the subvolume is over-sampled during diastole, and as a result volume flow is under-estimated due to the inclusion of excessive diastolic data.

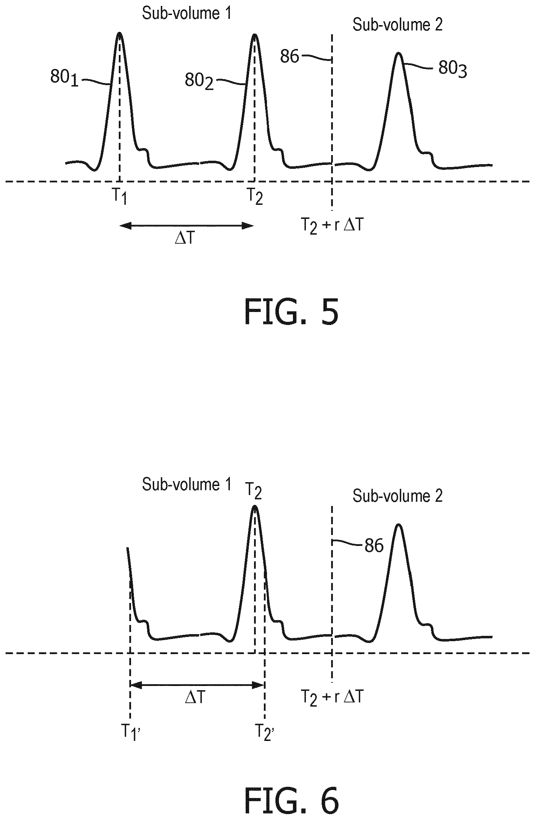

It is thus seen that accurate heart rate information is important for accurate volume flow assessment. In accordance with a further aspect of the present invention, when the heart rate is determined from ultrasonic signal information, the heart rate estimation is continuously updated during each subvolume acquisition to properly adjust for the occurrence of a longer or shorter interval between heartbeats. A heartrate estimation can be estimated from M-mode data as described in US Pat. 9,357,978 (Dow et al.) This can be done in the background, with the heartrate estimated even before volume flow acquisition begins, thereby enabling the acquisition of the first subvolume in proper synchrony with the heartrate. Another way to determine the heartrate is to continuously calculate the flow profile signal during each subvolume acquisition, and use this updated information to properly synchronize the following acquisition with the phase of the heart. One example of this technique is illustrated in FIGURE 5. In this example the first subvolume is acquired over two heart cycles, the first exhibiting a systolic flow phase $80_1$ which peaks at time $T_1$ and the second exhibiting a systolic flow phase $80_2$ which peaks at time $T_2$. The two systolic peaks are detected by peak detection of the flow profile signal, and the peak-to-peak interval $\Delta T$ is determined. The time of commencement of acquisition of the second subvolume is then set as a function of the $\Delta T$ interval and the time of occurrence of $T_2$. Preferably the fraction of the $\Delta T$ interval is one-half, which causes acquisition of the second subvolume to start midway in the diastolic phase. Thus, calculating $T_2 + r\Delta T$ when r is set to 0.5 will cause acquisition of the subsequent subvolume to commence in the middle of the diastolic phase and result in sampling the next subvolume over the interval of waveform $80_3$ in FIGURE 5. This timing will advantageously result in sampling of the second subvolume with its systolic phase occurring during the middle of the acquisition interval, assuring that all of the systolic phase will be sampled.

FIGURE 6 provides a second example of use of the flow profile waveform to establish subvolume acquisition timing. In this example acquisition of subvolume 1 starts at $T_1'$, just after a systolic peak of the flow signal. The same level of the next systolic phase is detected, which occurs at time $T_2'$, and the interval $\Delta T$ between $T_1'$ and $T_2'$ is determined. The equation $T_2 + r\Delta T$ is calculated which yields a starting time for acquiring the second subvolume as shown at 86. While the first subvolume is not sampled with the systolic phase temporally located in the middle of the subvolume acquisition interval, properly phased acquisition will occur for the second and all subsequent subvolumes.

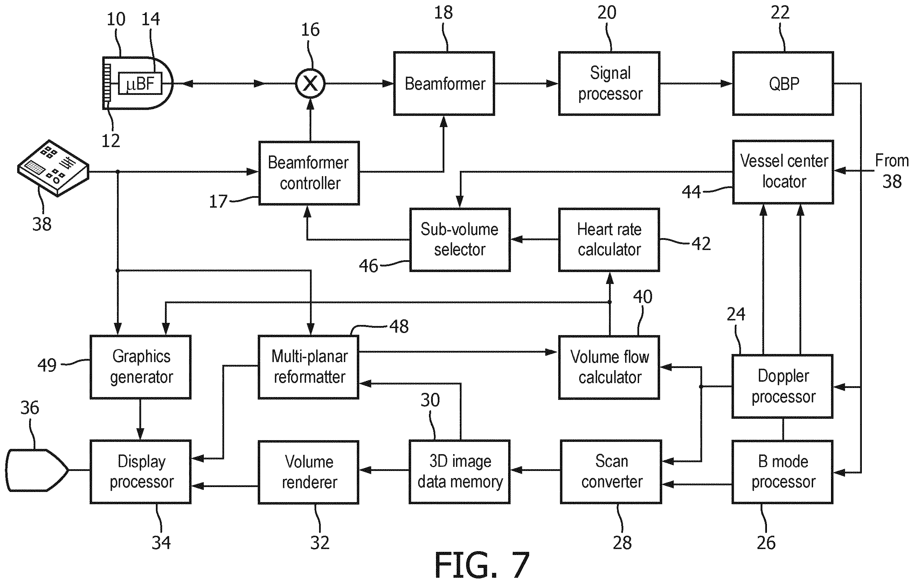

In FIGURE 7, an ultrasound system constructed in accordance with the principles of the present invention is shown in block diagram form. A transducer array 12 is provided in an ultrasound probe 10 for transmitting ultrasonic waves and receiving echo information. The transducer array 12 is an array of transducer elements capable of scanning in three dimensions, in both elevation and azimuth. The transducer array 12 is coupled to a microbeamformer 14 in the probe which controls transmission and reception of signals by the array elements. Microbeamformers are capable of at least partial beamforming of the signals received by groups or "patches" of transducer elements as described in US Pats. 5,997,479 (Savord et al.), 6,013,032 (Savord), and 6,623,432 (Powers et al.) The microbeamformer is coupled by the probe cable to a transmit/receive (T/R) switch 16 which switches between transmission and reception and protects the main beamformer 18 from high energy transmit signals. The transmission of ultrasonic beams from the transducer array 12 under control of the microbeamformer 14 is directed by a beamformer controller 17 coupled to the T/R switch and the main beamformer 18, which receives input from the user's operation of the user interface or control panel 38. Among the transmit characteristics controlled by the transmit controller are the direction, number, spacing, amplitude, phase, frequency, polarity, and diversity of transmit waveforms. Beams formed in the direction of pulse transmission may be steered straight ahead from the transducer array, or at different angles on either side of an unsteered beam for a wider sector field of view.

The echoes received by a contiguous group of transducer elements are beamformed by appropriately delaying them and then combining them. The partially beamformed signals produced by the microbeamformer 14 from each patch are coupled to the main beamformer 18 where partially beamformed signals from individual patches of transducer elements are combined into a fully beamformed coherent echo signal. For example, the main beamformer 18 may have 128 channels, each of which receives a partially beamformed signal from a patch of 12 transducer elements. In this way the signals received by over 1500 transducer elements of a two-dimensional matrix array transducer can contribute efficiently to a single beamformed signal.

The coherent echo signals undergo signal processing by a signal processor 20, which includes filtering by a digital filter and noise reduction as by spatial or frequency compounding. The signal processor may also perform speckle reduction as by spatial or frequency compounding. The digital filter of the signal processor 20 can be a filter of the type disclosed in U.S. Patent No. 5,833,613 (Averkiou et al.), for example. The echo signals are then coupled to a quadrature bandpass filter (QBP) 22. The QBP performs three functions: band limiting the r.f. echo signal data, producing in-phase and quadrature pairs (I and Q) of echo signal data, and decimating the digital sample rate. The QBP comprises two separate filters, one producing in-phase samples and the other producing quadrature samples, with each filter being formed by a plurality of multiplier-accumulators (MACs) implementing an FIR filter.

The beamformed and processed coherent echo signals are coupled to a pair of image data processors. A B mode processor 26 produces signal data for a B mode image of structure in the body such as tissue. The B mode processor performs amplitude (envelope) detection of quadrature demodulated I and Q signal components by calculating the echo signal amplitude in the form of $(I^2+Q^2)^{1/2}$. The quadrature echo signal components are also coupled to a Doppler processor 24. The Doppler processor 24 stores ensembles of echo signals from discrete points in an image field which are then used to estimate the Doppler shift at points in the image with a fast Fourier transform (FFT) processor. The rate at which the ensembles are acquired determines the velocity range of motion that the system can accurately measure and depict in an image. The Doppler shift is proportional to motion at points in the image field, e.g., blood flow and tissue motion. For color Doppler image data, the estimated Doppler flow values at each point in a blood vessel are wall filtered and converted to color values using a look-up table. The wall filter has an adjustable cutoff frequency above or below which motion will be rejected such as the low frequency motion of the wall of a blood vessel when imaging flowing blood. The B mode image data and the Doppler flow values are coupled to a scan converter 28 which converts the B mode and Doppler samples from their acquired R-θ coordinates to Cartesian (x,y) coordinates for display in a desired display format, e.g., a rectilinear display format or a sector display format. Either the B mode image or the Doppler image may be displayed alone, or the two shown together in anatomical registration in which the color Doppler overlay shows the blood flow in B mode processed tissue and vessels in the image. Another display possibility is to display side-by-side images of the same anatomy which have been processed differently. This display format is useful when comparing images. The scan-converted image data, both B mode and Doppler data, is coupled to and stored in a 3D image data memory 30 where it is stored in memory locations addressable in accordance with the spatial locations from which the image data values were acquired. Image data from 3D scanning can be accessed by a volume renderer 32, which converts the data values of a 3D data set into a projected 3D image as viewed from a given reference point as described in US Pat. 6,530,885 (Entrekin et al.) The 3D images produced by the volume renderer 32 and 2D images from data produced by the scan converter 28 are coupled to a display processor 34 for further enhancement, buffering and temporary storage for display on an image display 36. The 3D image data is also coupled to a multi-planar reformatter 48 which, in response to user input from the user controls 38, is able to extract image data for a user-designated image plane from the 3D dataset. This image data is coupled to the display processor 34 for display of a selected MPR image, and the plane of the MPR image is used in the estimation of volume flow as described below.

In accordance with the present invention, B mode and Doppler data produced by processors 24 and 26 are coupled to a vessel center locator 44. This enables the vessels center locator to do several things. One is to enable a user to click on a point in a B mode image of a blood vessel which the user believes is the center of the vessel. A signal indicating this user action is coupled to the vessel center locator from the user interface 38, and the identified vessel center point is stored in the locator and coupled to subvolume selector 46. Another operation of the vessel center locator 44 is to receive velocity data from the Doppler processor after a rapid Doppler scan of a blood vessel. The locator 44 analyzes this data to determine the spatial location in the vessel with the highest flow velocity. In that case, this spatial location is coupled to the subvolume selector 46 and used as the vessel center. It will be appreciated that the B mode and Doppler data coupled to the vessel center locator can be that which is processed by scan conversion, so that the spatial location coordinates will correspond with that used by the display 36. The vessel center locator 44 is thus capable of using either user input or automated methods to determine a vessel center and couple that information to the subvolume selector 46.

The multi-planar reformatter 48 is also coupled to a volume flow calculator 40. The volume flow calculator also receives Doppler velocity data from the Doppler processor 24 and is thus able to compute the volume blood flow through a B- or C-plane of a blood vessel using Gauss's theorem. For volume flow data, Gauss's theorem is calculated as:

$$Q = \int_S v \cdot \mathrm{d}A$$

where Q is the volume flow in, e.g., milliliters per second, *v* is flow velocity, and the surface *S* is a selected plane through a vessel lumen. A surface integral of velocity v over the enclosing boundary *S* yields the volume flow Q. Volume flow through a plane intersecting a blood vessel can thus be updated with new data for each new phase of the heart cycle to produce a flow profile curve of Q as a function of time, and the flow volume over the phases of an entire heart cycle can be summed to calculate the volume flow per heart cycle.

The flow data of volume flow produced by the volume flow calculator 40 is coupled to a graphics generator 49, which produces a flow profile curve such as that shown in FIGURE 4 for display on the display 36. The graphics generator also produces graphics for display with the ultrasound image for things such as cursors, measurement dimensions, exam parameters, and patient name.

The flow profile curve data is also coupled to a heart rate calculator 42, where it is used to estimate the heartrate in the absence of ECG monitor signals or user input of a heartrate value. The heartrate calculator uses the flow profile curve data to detect systolic peaks of the flow profile, to detect the interval ΔT between systolic peaks, and to calculate the start times for successive subvolume acquisitions as described above. The heartrate timing data is coupled to the subvolume selector 46, which determines when to acquire each subvolume needed to scan the entire volumetric region of a vessel. The data from the vessel center locator 44 informs the subvolume selector of where the first subvolume is to be acquired (*i.e.,* around the vessel center,) and the data from the heart rate calculator 42 informs the subvolume selector of the timing of each subvolume acquisition so that the systolic phase will be acquired in the middle of each subvolume acquisition for at least the second and subsequent subvolume acquisitions. Acting on this information, the subvolume selector informs the beamformer controller of when and where each subvolume acquisition is to be performed. The ultrasound system of FIGURE 7 thereby performs acquisition and assembly of a volume image of volume flow data for the accurate estimation of a volume flow profile curve.

**Claims**

1. An ultrasonic diagnostic imaging system for analyzing volume flow of blood comprising:

   a 3D imaging probe (10) adapted to acquire volume image flow data sets of a blood vessel;
   an image data processor (24, 26) responsive to the volume image flow data sets;
   a vessel center locator (44), responsive to spatially organized blood vessel data, which is adapted to identify a center of the blood vessel;
   a beamformer controller (17), responsive to the vessel center locator (44), which is adapted to control the 3D imaging probe (10) to acquire volume image flow data sets of the blood vessel starting with a subvolume (101) from the center of the vessel ;
   a volume flow calculator (40), responsive to acquired volume image flow data sets of the blood vessel, which is adapted to calculate volume flow profile data; and
   a heart rate calculator (42) adapted to produce data representing a heart rate, wherein the volume image flow data sets are acquired in timed relation to the heart rate data,
   wherein the heart rate calculator (42) is coupled to the volume flow calculator (40) and adapted to calculate a subvolume acquisition time (86) in relation to systolic peaks of a flow profile (80).

2. The ultrasonic diagnostic imaging system of Claim 1, wherein the heart rate calculator (42) further comprises one of an ECG monitor or an ultrasound data processor which is adapted to produce estimated heart rate data using ultrasound data.

3. The ultrasonic diagnostic imaging system of Claim 1, further comprising a subvolume selector (46) responsive to the vessel center locator which is adapted to control the beamformer controller (17) to acquire volume image flow data sets of subvolumes (101-103) of a blood vessel starting with a subvolume (101) from the center of the vessel.

4. The ultrasonic diagnostic imaging system of Claim 3, wherein the beamformer controller (17) is further adapted to acquire volume image flow data sets of a subvolume (101-103) of a blood vessel over the duration of a heart cycle.

5. The ultrasonic diagnostic imaging system of Claim 4, wherein the beamformer controller (17) is further adapted to acquire volume image flow data sets of a subvolume (101-103) of a blood vessel over an acquisition interval commencing in the middle of a diastolic portion of a heart cycle and ending in the middle of the next diastolic portion of a heart cycle.

6. The ultrasonic diagnostic imaging system of Claim 5, wherein the beamformer controller (17) is further adapted to acquire volume image flow data sets of a subvolume (101-103) of a blood vessel during systolic heart phases occurring in the middle of the acquisition interval.

7. The ultrasonic diagnostic imaging system of Claim 1, further comprising:

   a 3D image data memory (30) adapted to store volume image data sets; and
   a multi-planar reformatter (48), coupled to the 3D image data memory (30), and adapted to select an image plane intersecting the blood vessel,
   wherein the volume flow calculator 40) is further adapted to calculate volume flow profile data in relation to the image plane intersecting the blood vessel.

8. The ultrasonic diagnostic imaging system of Claim 7, further comprising a volume renderer (32) coupled to the 3D image data memory (30) and adapted to produce a 3D image of the blood vessel.

9. The ultrasonic diagnostic imaging system of Claim 8, further comprising a display (36), coupled to the multi-planar reformatter (48), the volume renderer (32), and the volume flow calculator (40), which is adapted to display one or more of an image plane selected by the multi-planar reformatter (48), a 3D image of the blood vessel, and a flow profile curve.

10. A method of analyzing volume flow of blood by ultrasound data acquisition comprising:

    identifying a center of a blood vessel;
    estimating heart cycle timing;
    acquiring, with a 3D ultrasound imaging probe (10), volume image flow data sets of the blood vessel from subvolumes (101-103) of the vessel starting with a subvolume (101) from the center of the vessel and in synchronism with the heart cycle timing, wherein a subvolume acquisition time (86) is calculated in relation to systolic peaks of a flow profile (80);
    processing the volume image flow data sets acquired with the 3D ultrasound imaging probe (10) for the display of an ultrasound image of the blood vessel; and
    calculating volume flow profile data using the acquired volume image flow data sets.

11. The method of Claim 10, further comprising:

    detecting a systolic phase of the heart cycle;
    wherein acquiring volume image flow data sets further comprises acquiring volume image flow data sets of the blood vessel from a subvolume (101-103) of the vessel during an acquisition interval starting in mid-diastole of a heart cycle and ending in mid-diastole of a subsequent heart cycle,
    wherein acquisition during the systolic phase occurs during the middle of the acquisition interval.

12. The method of Claim 11,
    wherein acquiring volume image flow data sets of the blood vessel from subvolumes (101-103) of the vessel further comprises updating the estimated heart cycle timing during a plurality of the subvolume acquisition intervals.

**Patentansprüche**

1. Ultraschall-Diagnosebildgebungssystem zur Analyse des Volumenstroms von Blut, umfassend:

   eine 3D-Bildgebungssonde (10), die angepasst ist, um Volumenbildstromdatensätze eines Blutgefäßes zu erfassen;
   einen Bilddatenprozessor (24, 26), der auf die Volumenbildstromdatensätze reagiert;
   einen Gefäßmitten-Positionsgeber (44), der auf räumlich organisierte Blutgefäßdaten reagiert, der angepasst ist, um eine Mitte des Blutgefäßes zu identifizieren;
   eine Strahlformer-Steuereinheit (17), die auf den Gefäßmitten-Positionsgeber (44) reagiert, die angepasst ist, um die 3D-Bildgebungssonde (10) zu steuern, um Volumenbildstromdatensätze des Blutgefäßes beginnend mit einem Teilvolumen (101) von der Mitte des Gefäßes aus zu erfassen;
   einen Volumenstromrechner (40), der auf erfasste Volumenbildstromdatensätze des Blutgefäßes reagiert, der angepasst ist, um Volumenstromprofildaten zu berechnen; und
   einen Herzfrequenzrechner (42), der angepasst ist, um Daten zu erzeugen, die eine Herzfrequenz darstellen, wobei die Volumenbildstromdatensätze in zeitlicher Beziehung zu den Herzfrequenzdaten erfasst werden,
   wobei der Herzfrequenzrechner (42) mit dem Volumenstromrechner (40) gekoppelt ist und angepasst ist, um eine Teilvolumenerfassungszeit (86) in Bezug auf systolische Spitzen eines Stromprofils (80) zu berechnen.

2. Ultraschall-Diagnosebildgebungssystem nach Anspruch 1, wobei der Herzfrequenzrechner (42) weiter einen von einem EKG-Monitor oder einen Ultraschalldatenprozessor umfasst, der angepasst ist, um unter Verwendung von Ultraschalldaten geschätzte Herzfrequenzdaten zu erzeugen.

3. Ultraschall-Diagnosebildgebungssystem nach Anspruch 1, das weiter einen Teilvolumenselektor (46) umfasst, der auf den Gefäßmitten-Positionsgeber reagiert und angepasst ist, um die Strahlformer-Steuereinheit (17) zu steuern, um Volumenbildstromdatensätze von Teilvolumina (101-103) eines Blutgefäßes beginnend mit einem Teilvolumen (101) von der Mitte des Gefäßes aus zu erfassen.

4. Ultraschall-Diagnosebildgebungssystem nach Anspruch 3, wobei die Strahlformer-Steuereinheit (17) weiter angepasst ist, um Volumenbildstromdatensätze eines Teilvolumens (101-103) eines Blutgefäßes über die Dauer eines Herzzyklus hinweg zu erfassen.

5. Ultraschall-Diagnosebildgebungssystem nach Anspruch 4, wobei die Strahlformer-Steuereinheit (17) weiter angepasst ist, um Volumenbildstromdatensätze eines Teilvolumens (101-103) eines Blutgefäßes über ein Erfassungsintervall zu erfassen, das im Mittelteil eines diastolischen Abschnitts eines Herzzyklus beginnt und im Mittelteil des nächsten diastolischen Abschnitts eines Herzzyklus endet.

6. Ultraschall-Diagnosebildgebungssystem nach Anspruch 5, wobei die Strahlformer-Steuereinheit (17) weiter angepasst ist, um Volumenbildstromdatensätze eines Teilvolumens (101-103) eines Blutgefäßes während systolischer Herzphasen zu erfassen, die in dem Mittelteil des Erfassungsintervalls auftreten.

7. Ultraschall-Diagnosebildgebungssystem nach Anspruch 1, das weiter umfasst:

   einen 3D-Bilddatenspeicher (30), der angepasst ist, um Volumenbilddatensätze zu speichern; und
   einen multiplanaren Umformatierer (48), der mit dem 3D-Bilddatenspeicher (30) gekoppelt ist und angepasst ist, um eine das Blutgefäß schneidende Bildebene auszuwählen, wobei der Volumenstromrechner (40) weiter angepasst ist, um Volumenstromprofildaten in Bezug auf die das Blutgefäß schneidende Bildebene zu berechnen.

8. Ultraschall-Diagnosebildgebungssystem nach Anspruch 7, das weiter einen Volumenrenderer (32) umfasst, der mit dem 3D-Bilddatenspeicher (30) gekoppelt ist und angepasst ist, um ein 3D-Bild des Blutgefäßes zu erzeugen.

9. Ultraschall-Diagnosebildgebungssystem nach Anspruch 8, das weiter eine Anzeige (36) umfasst, die mit dem multiplanaren Umformatierer (48), dem Volumenrenderer (32) und dem Volumenstromrechner (40) gekoppelt ist, die angepasst ist, um eines oder mehr von einer Bildebene, die von dem multiplanaren Umformatierer (48) ausgewählt ist, einem 3D-Bild des Blutgefäßes und einer Stromprofilkurve anzuzeigen.

10. Verfahren zur Analyse des Volumenstroms von Blut durch Ultraschalldatenerfassung, umfassend:

    Identifizieren einer Mitte eines Blutgefäßes;
    Schätzen des Herzzyklus-Timings;
    Erfassen mit einer 3D-Ultraschall-Bildgebungssonde (10) von Volumenbildstromdatensätzen des Blutgefäßes aus Teilvolumina (101-103) des Gefäßes, beginnend mit einem Teilvolumen (101) von der Mitte des Gefäßes aus und synchron mit dem Herzzyklus-Timing, wobei eine Teilvolumenerfassungszeit (86) in Bezug auf systolische Spitzen eines Stromprofils (80) berechnet wird;
    Verarbeiten der mit der 3D-Ultraschallbildgebungssonde (10) erfassten Volumenbildstromdatensätze zur Anzeige eines Ultraschallbildes des Blutgefäßes; und
    Berechnen von Volumenstromprofildaten unter Verwendung der erfassten Volumenbildstromdatensätze.

11. Verfahren nach Anspruch 10, weiter umfassend:

    Erkennen einer systolischen Phase des Herzzyklus;
    wobei Erfassen von Volumenbildstromdatensätzen weiter Erfassen von Volumenbildstromdatensätzen des Blutgefäßes aus einem Teilvolumen (101-103) des Gefäßes während eines Erfassungsintervalls beginnend bei einer halben Diastole eines Herzzyklus und bei einer halben Diastole eines nachfolgenden Herzzyklus endend umfasst, wobei Erfassung während der systolischen Phase in dem Mittelteil des Erfassungsintervalls erfolgt.

12. Verfahren nach Anspruch 11, wobei Erfassen von Volumenbildstromdatensätzen des Blutgefäßes aus Teilvolumina (101-103) des Gefäßes weiter Aktualisieren des geschätzten Herzzyklus-Timings während einer Vielzahl von Teilvolumenerfassungsintervallen umfasst.

**Revendications**

1. Système d'imagerie diagnostique ultrasonore pour analyser un débit volumique du sang comprenant :

   une sonde d'imagerie 3D (10) conçue pour acquérir des ensembles de données de flux d'image de volume d'un vaisseau sanguin ;
   un processeur de données d'image (24, 26) sensible aux ensembles de données de flux d'image de volume ;
   un localisateur de centre de vaisseau (44), sensible à des données de vaisseau sanguin organisées spatialement, qui est conçu pour identifier un centre du vaisseau sanguin ;
   un dispositif de commande (17) de formeur de faisceaux, sensible au localisateur de centre de vaisseau (44), qui est conçu pour commander la sonde d'imagerie 3D (10) pour acquérir des ensembles de données de flux d'image de volume du vaisseau sanguin en commençant par un sous-volume (101) à partir du centre du vaisseau ;
   un calculateur de débit volumique (40), sensible aux ensembles de données de flux d'image de volume acquis du vaisseau sanguin, qui est conçu pour calculer des données de profil de débit volumique ; et
   un calculateur de fréquence cardiaque (42) conçu pour produire des données représentant une fréquence cardiaque, dans lequel les ensembles de données de flux d'image de volume sont acquis de manière synchronisée avec les données de fréquence cardiaque,
   dans lequel le calculateur de fréquence cardiaque (42) est couplé au calculateur de débit volumique (40) et conçu pour calculer un temps d'acquisition de sous-volume (86) par rapport à des pics systoliques d'un profil de débit (80).

2. Système d'imagerie diagnostique ultrasonore selon la revendication 1, dans lequel le calculateur de fréquence cardiaque (42) comprend en outre l'un parmi un moniteur ECG ou un processeur de données ultrasonores qui est conçu pour produire des données de fréquence cardiaque estimées à l'aide de données ultrasonores.

3. Système d'imagerie diagnostique ultrasonore selon la revendication 1, comprenant en outre un sélecteur de sous-volume (46) sensible au localisateur de centre de vaisseau qui est conçu pour commander le dispositif de commande (17) de formeur de faisceaux pour acquérir des ensembles de données de flux d'image de volume de sous-volumes (101-103) d'un vaisseau sanguin en commençant par un sous-volume (101) à partir du centre du vaisseau.

4. Système d'imagerie diagnostique ultrasonore selon la revendication 3, dans lequel le dispositif de commande (17) de formeur de faisceaux est en outre conçu pour acquérir des ensembles de données de flux d'image de volume d'un sous-volume (101-103) d'un vaisseau sanguin pendant la durée d'un cycle cardiaque.

5. Système d'imagerie diagnostique ultrasonore selon la revendication 4, dans lequel le dispositif de commande (17) de formeur de faisceaux est en outre conçu pour acquérir des ensembles de données de flux d'image de volume d'un sous-volume (101-103) d'un vaisseau sanguin pendant un intervalle d'acquisition commençant au milieu d'une partie diastolique d'un cycle cardiaque et se terminant au milieu de la partie diastolique suivante d'un cycle cardiaque.

6. Système d'imagerie diagnostique ultrasonore selon la revendication 5, dans lequel le dispositif de commande (17) de formeur de faisceaux est en outre conçu pour acquérir des ensembles de données de flux d'image de volume d'un sous-volume (101-103) d'un vaisseau sanguin pendant des phases cardiaques systoliques se produisant au milieu de l'intervalle d'acquisition.

7. Système d'imagerie diagnostique ultrasonore selon la revendication 1, comprenant en outre :

   une mémoire de données d'image 3D (30) conçue pour stocker des ensembles de données d'image de volume ; et
   un reformateur multiplanaire (48), couplé à la mémoire de données d'image 3D (30), et conçu pour sélectionner un plan d'image coupant le vaisseau sanguin, dans lequel le calculateur de débit volumique (40) est en outre conçu pour calculer des données de profil de débit volumique par rapport au plan d'image coupant le vaisseau sanguin.

8. Système d'imagerie diagnostique ultrasonore selon la revendication 7, comprenant en outre un moteur de rendu de volume (32) couplé à la mémoire de données d'image 3D (30) et conçu pour produire une image 3D du vaisseau sanguin.

9. Système d'imagerie diagnostique ultrasonore selon la revendication 8, comprenant en outre un écran (36), couplé au reformateur multiplanaire (48), au moteur de rendu de volume (32) et au calculateur de débit volumique (40), qui est conçu pour afficher un ou plusieurs d'un plan d'image sélectionné par le reformateur multiplanaire (48), d'une image 3D du vaisseau sanguin et d'une courbe de profil de débit.

**10.** Procédé d'analyse d'un débit volumique du sang par acquisition de données ultrasonores comprenant :

l'identification d'un centre d'un vaisseau sanguin ; l'estimation d'une durée de cycle cardiaque ;

l'acquisition, avec une sonde d'imagerie ultrasonore 3D (10), d'ensembles de données de flux d'image de volume du vaisseau sanguin à partir de sous-volumes (101-103) du vaisseau en commençant par un sous-volume (101) à partir du centre du vaisseau et en synchronisme avec la durée de cycle cardiaque,

dans lequel un temps d'acquisition de sous-volume (86) est calculé par rapport à des pics systoliques d'un profil de débit (80) ;

le traitement des ensembles de données de flux d'image de volume acquis avec la sonde d'imagerie à ultrasons 3D (10) pour l'affichage d'une image ultrasonore du vaisseau sanguin ; et

le calcul de données de profil de débit volumique à l'aide des ensembles de données de flux d'image de volume acquis.

**11.** Procédé selon la revendication 10, comprenant en outre :

la détection d'une phase systolique du cycle cardiaque ;

dans lequel l'acquisition d'ensembles de données de flux d'image de volume comprend en outre l'acquisition d'ensembles de données de flux d'image de volume du vaisseau sanguin à partir d'un sous-volume (101-103) du vaisseau pendant un intervalle d'acquisition commençant en mi-diastole d'un cycle cardiaque et se terminant en mi-diastole d'un cycle cardiaque ultérieur, dans lequel l'acquisition pendant la phase systolique se produit au milieu de l'intervalle d'acquisition.

**12.** Procédé selon la revendication 11 :

dans lequel l'acquisition d'ensembles de données de flux d'image de volume du vaisseau sanguin à partir de sous-volumes (101-103) du vaisseau comprend en outre la mise à jour de la durée estimée de cycle cardiaque pendant une pluralité d'intervalles d'acquisition de sous-volumes.

FIG. 1

Sub-volume 5

Sub-volume 3 ——— 103

Sub-volume 1 ——— 101

Sub-volume 2 ——— 100 ——— 102

Sub-volume 4

# FIG. 2

Sub-volume 1    Sub-volume 2    Sub-volume 3

Sub-volume 4

Sub-volume 5

81    82    83    84    85

# FIG. 3

FIG. 4

Sub-volume 1

Sub-volume 2

$80_1$

$80_2$

$86$

$80_3$

$T_1$ $T_2$ $T_2 + r \Delta T$

$\Delta T$

# FIG. 5

Sub-volume 1 $T_2$

Sub-volume 2

$86$

$\Delta T$

$T_2 + r \Delta T$

$T_{1'}$ $T_{2'}$

# FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2008287799 A1
- US 6503202 B1
- WO 2019156975 A1
- US 9357978 B, Dow
- US 5997479 A, Savord
- US 6013032 A, Savord
- US 6623432 B, Powers
- US 5833613 A, Averkiou
- US 6530885 B, Entrekin